Europäisches Patentamt

European Patent Office

Office européen des brevets

(19)

(11) Veröffentlichungsnummer : **0 105 428**
**B1**

(12) **EUROPÄISCHE PATENTSCHRIFT**

(45) Veröffentlichungstag der Patentschrift :
04.03.87

(21) Anmeldenummer : 83109521.1

(22) Anmeldetag : 24.09.83

(51) Int. Cl.⁴ : **A 61 F 13/20**

(54) **Tampon für die Frauenhygiene und Verfahren zu dessen Herstellung.**

(30) Priorität : 02.10.82 DE 3236540

(43) Veröffentlichungstag der Anmeldung :
18.04.84 Patentblatt 84/16

(45) Bekanntmachung des Hinweises auf die Patenterteilung : 04.03.87 Patentblatt 87/10

(84) Benannte Vertragsstaaten :
AT BE CH DE FR GB IT LI NL SE

(56) Entgegenhaltungen :
DE-A- 1 491 235
DE-A- 2 855 179
GB-A-    594 061
GB-A-    844 676
GB-A-    855 119
US-A- 3 051 177

(73) Patentinhaber : **Vereinigte Papierwerke AG**
**Schoppershofstrasse 80**
**D-8500 Nürnberg (DE)**

(72) Erfinder : **Sustmann, Scarlet, Dr.**
**Am Nachtigallenwäldchen 34**
**D-4060 Viersen 12 (DE)**

(74) Vertreter : **Pohl, Hans Ludwig**
**Hefnersplatz 3**
**D-8500 Nürnberg 11 (DE)**

Jouve, 18, rue St-Denis. 75001 Paris, France

## Beschreibung

Die Erfindung betrifft ein Verfahren zum Herstellen von Tampons für die Frauenhygiene, bei dem Watteband mit einem flusenfreien Hüllmaterial überlappend umhüllt wird, das Hüllmaterial und das Watteband, zur Bildung von umhüllten Wattebandabschnitten mit freien Schnittkanten, senkrecht zur Überlappungslinie des Hüllmaterials durchtrennt werden und jeder umhüllte Wattebandabschnitt mit einem parallel oder senkrecht zur Überlappungslinie aufgebrachten Rückholfaden ausgestattet und mittels Rundbacken mit Bezug auf die Überlappungslinie radial oder axial in W-förmiger Faltung verpreßt wird.

Bei bisher verwendeten Tampons können Fasern bzw. Wattebestandteile in der Vagina zurückbleiben. Man hat daher versucht, den faserförmigen Wattebandabschnitt beim Herstellen von Tampons flusendicht zu umhüllen. Eine entsprechende Umhüllung kann z. B. aus Rayon, Synthesefasern oder aus einem beliebigen Gemisch dieser Materialien, vorzugsweise als Nonwoven, bestehen. Beispielsweise werden Tampons nach einem Verfahren hergestellt, bei dem der rechteckige Wattebandabschnitt in Nonwoven eingeschlagen und der zugehörige Rückholfaden bzw. die Rückholkordel in Längsrichtung des Wattebands aufgenäht wird. Das Verpressen kann dann mit Bezug auf die Längsrichtung des Wattebandes radial und/oder axial erfolgen. Bei der Radialpressung wird eine W-förmige Faltung, d. h. eine Faltung mit Vierfachlage, bevorzugt. Das Verfahren läßt jedoch ein Umhüllen des Tampons an den Schnittkanten, d. h. an Tamponkopf und Tamponende, nicht zu, so daß sich hier Fasern ablösen — d. h. vom Watteband aus nach außen dringen — können.

Bei einem anderen zum Herstellen von Tampons angewendeten Verfahren wird das Watteband in Nonwoven eingeschlagen und dann zu rechteckigen Abschnitten geschnitten. Dabei wird die Länge des zu verpressenden Abschnitts durch die Breite des Wattebandes vorgegeben. Der Rückholfaden wird also nicht parallel sondern senkrecht zu der Überlapplinie des Hüllmaterials aufgesetzt. Auch bei diesem Verfahren wird eine W-förmige Faltung des umhüllten Wattebandabschnitts vorgezogen, wobei jedoch die Faltlinien parallel zu den freien Schnittkanten liegen. Dadurch wird erreicht, daß die freien Schnittkanten von Kopf und Ende des Tampons auf dessen im wesentlichen zylindrische Umfangsfläche verlegt sind ; dort liegen die Kanten aber nach wie vor offen. Insbesondere nach Ausdehnung des Tampons bei Flüssigkeitsaufnahme können daher beim Entnehmen Fasern von den Schnittkanten abgezogen werden.

Aus GB-A-59 40 61 ist ein Verfahren der eingangs genannten Art zum Herstellen von Tampons bekannt, wobei die Schnittkanten eines mit Vlies bedeckten bzw. eingehüllten Wattebandstückes aufeinander gefaltet und dann zusammengenäht werden. Schließlich wird das so hergestellte Zwischenerzeugnis über die Naht umgestülpt, dergestalt, daß die Schnittkanten innerhalb des Gebildes liegen. Für die Durchführung dieser Maßnahmen ist eine komplizierte Technik erforderlich, welche sich für eine rationelle Massenfertigung verbietet.

Weiterhin ist aus DE-A-28 55 179 das Einschlagen versiegelter Enden bekannt, welche dann durch die spezielle Tamponherstellung — mittige Fadenumschlingung/Querfaltung — im Innern liegen. Das Versiegeln überstehender Vliesenden ist eine Maßnahme, welche eine Massenherstellung erschwert.

Der Erfindung liegt demgegenüber die Aufgabe zu Grunde, ein Verfahren anzugeben, womit vollständig mit einem flusenfreien Material umhüllte Tampons geschaffen werden können, wobei Fasermaterial bei Gebrauch nicht abgelöst werden kann. Die Aufgabe bezieht sich insbesondere darauf, durch spezielle Faltung eines umhüllten Wattebandabschnitts eine flusendichte Vollumhüllung der hergestellten Tampons zu gewährleisten.

Die erfindungsgemäße Lösung besteht in der Kombination der im Anspruch genannten Maßnahmen.

Durch die Erfindung wird eine Vollumhüllung des Tampons dadurch erreicht, daß die nach dem Umhüllen des Wattebandes mit einem flusenfreien (d. h. gegenüber dem Material des Wattebandes auch flusendichten) Material erzeugten freien Schnittkanten ebenfalls auf die Fläche des umhüllten Wattebands umgelegt und dort verpreßt werden. Bei dem Herstellungsverfahren ist dazu ein entsprechender Arbeitsgang einzuschalten, bei dem der abgetrennte und umhüllte Wattebandabschnitt in einer Richtung senkrecht zur Längsrichtung des Wattebandes auf einem Führungsband weiterläuft und dort mit Hilfe von Faltungsblechen an den freien Schnittkanten umgelegt und gepreßt wird. Alle übrigen Handhabungem beim Herstellen des Tampons können sich dann in üblicher Weise im wesentlichen unverändert anschließen.

Anhand der schematischen Darstellung von Ausführungsbeispielen werden Einzelheiten der Erfindung erläutert. Es zeigen :

Figuren 1 bis 4 den Herstellungsgang eines Tampons bis zum Umfalten der freien Schnittkanten ;

Figuren 5 bis 8 den Herstellungsgang beim Verpressen des Tampons bei parallel zu den freien Schnittkanten verlaufendem Rückholfaden ;

Figuren 9 bis 10 eine zusätzliche Faltung des umhüllten Wattebandabschnitts vor dem Verpressen ;

Figuren 11 bis 16 das Falten und Verpressen des Tampons bei Watteverstärkung am Kopf oder Ende ; und

Figuren 17 bis 19 das Herstellen eines Tampons mit Watteverstärkung am Kopf und Ende.

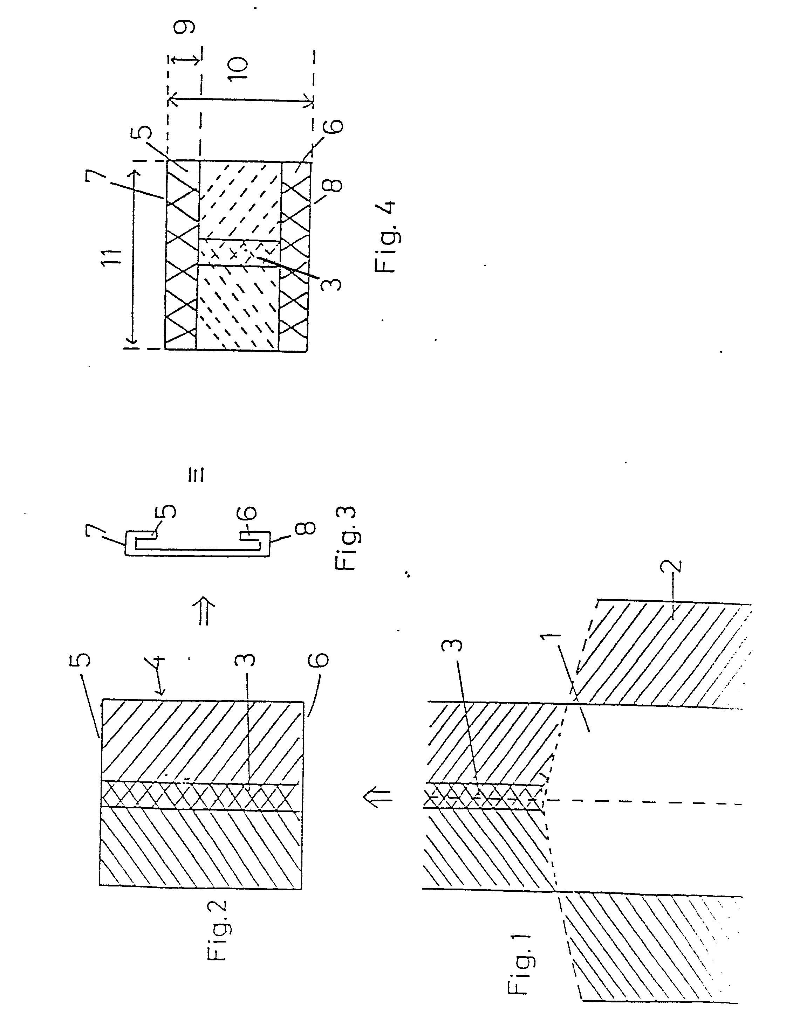

Gemäß Fig. 1 bis 4 wird in den in Pfeilrichtung aufeinanderfolgenden Herstellungsschritten ein Watteband 1 auf einem aus flusenfreiem sowie gegenüber Flusen des Wattebandes dichtem Material bestehenden Band 2 einer Faltstation zugeführt, in der das Band aus dem Hüllmaterial 2 so um das Watteband 1 geschlagen wird, daß es sich längs einer Überlapplinie 3 an den Längsrändern überdeckt. Das so umhüllte Watteband 1 gelangt dann zu einer Schneidstation, wo umhüllte Wattebandabschnitte 4 gemäß Fig. 2 mit freien Schnittkanten 5 und 6 abgetrennt werden. Das nicht flusende Hüllmaterial 2 soll ein relativ niedriges Flächengewicht, vorzugsweise weniger als 15 g/m², besitzen.

Die bis auf die freien Schnittkanten 5 und 6 flusenfrei umhüllten Wattebandabschnitte 4 laufen nun senkrecht zur bisherigen Transportrichtung in eine Einrichtung mit nicht gezeichneten Faltungsblechen, durch die ein Einschlagen der freien Schnittkanten 5 und 6 in der in Fig. 3 und 4 im Querschnitt und in der Draufsicht schematisch dargestellten Weise erreicht wird. Die in der Herstellungsphase nach Fig. 1 entstandene Überlapplinie 3 liegt dann etwa senkrecht zu den durch das Umlegen der freien Schnittkanten 5 und 6 entstandenen Faltlinien 7 und 8. Die Faltlinien 7 und 8 werden durch eine Kalanderwalze angepreßt. Die Größe des Einschlags 9 kann beliebig gewählt werden. Der Einschlag kann z. B. bis zu einem Sechstel des Abstandes 10 der Faltlinien 7 und 8 betragen. Es ist aber u. a. auch eine Überlappung dieses Einschlags möglich. Je nach der Tiefe des Einschlags 9 und der weiteren Verarbeitung sind die übrigen Maße, nämlich der Abstand 10 der Faltungslinien 7 und 8 und die Länge 11 von Fig. 4 sowie das Flächengewicht des verwendeten Wattebandes 1, vorzugeben.

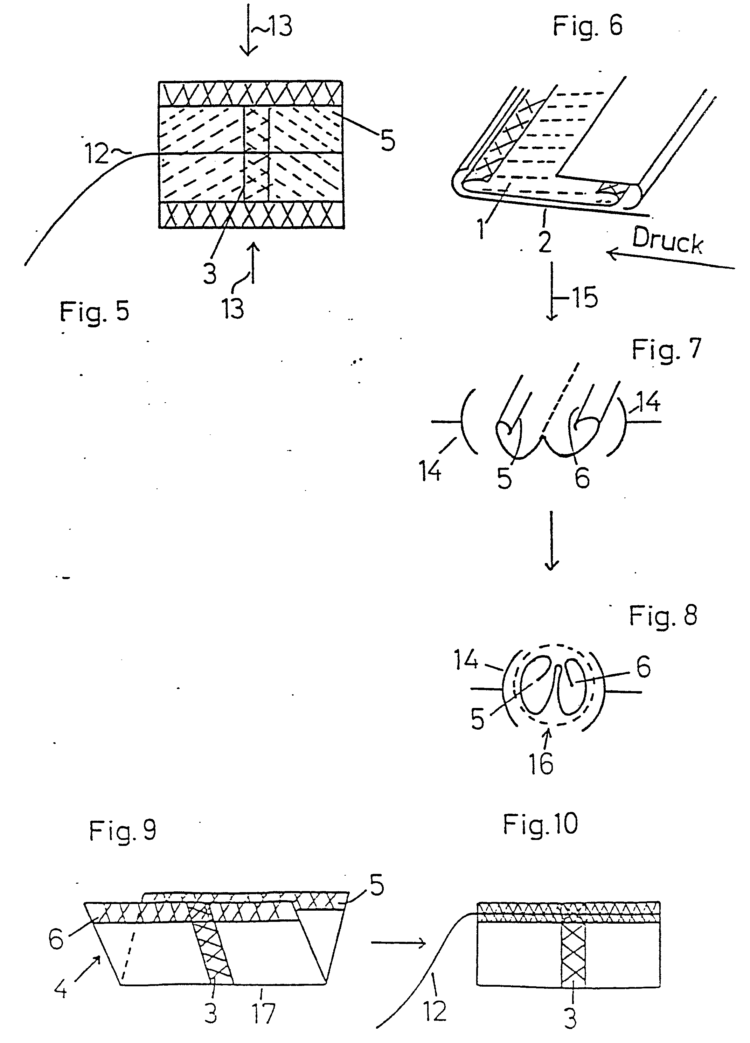

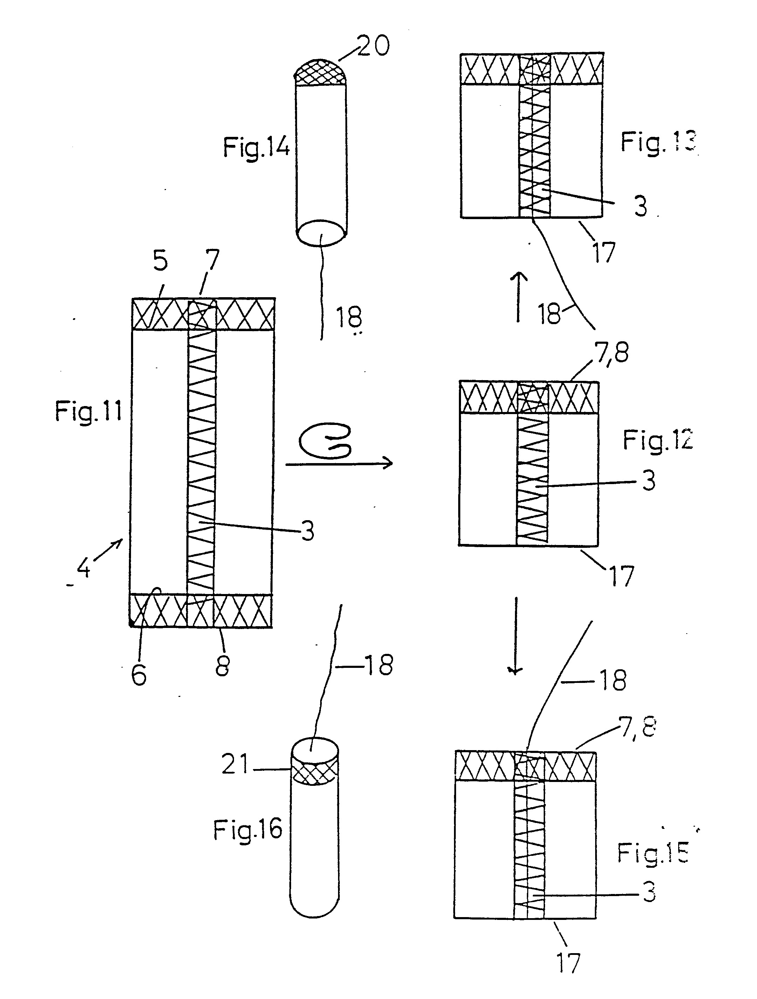

Für die weitere Verarbeitung sind verschiedene Möglichkeiten gegeben. Gemäß Fig. 5 bis 8 sowie 9 und 10 wird der Rückholfaden 12 parallel zu den Faltlinien 7 und 8 auf den umhüllten Wattebandabschnitt 4 aufgebracht, vorzugsweise aufgenäht. Gemäß Fig. 11 bis 19 wird der Rückholfaden 18 senkrecht zu den Faltlinien 7 und 8, also parallel zur Überlapplinie 3, auf den umhüllten Wattebandabschnitt 4 aufgebracht.

Bei dem Herstellungsverfahren nach Fig. 5 bis 8 wird der umhüllte und an den freien Schnittkanten 5 und 6 eingeschlagene sowie mit einem Rückholfaden 12 versehene Wattebandabschnitt 4 in Pfeilrichtung 13 in der in Fig. 5 bis 8 angedeuteten Weise, z. B. in einer Kammer mit Hilfe von zwei Rundbacken 14, verpreßt. Dabei entsteht eine W-förmige Faltung 15, in deren Inneren die freien Schnittkanten 5 und 6 liegen. Der entstehende rundgepreßte Tampon 16 wird in Fig. 8 im Querschnitt dargestellt. Anschließend kann der Tampon 16 in üblicher Weise auch in axialer Richtung — senkrecht zur Zeichnungsebene — verpreßt werden. Er ist an seinen mit den Vaginawänden in Berührung kommenden Flächen voll mit flusenfreiem bzw. flusendichtem Material umhüllt.

Eine weitere Möglichkeit des Faltens des Tampons wird in den Fig. 9 und 10 dargestellt. Hierbei wird der an den freien Schnittkanten 5 und 6 eingeschlagene umhüllte Wattebandabschnitt 4 vor dem Verpressen zusätzlich in Längsrichtung — um eine parallel zu den Schnittkanten 5 und 6 verlaufende Faltlinie 17 — nochmals gefaltet, so daß die Schnittkanten 5 und 6 innen liegen. Zum Fixieren der dann aufeinanderliegenden Flächen wird der Rückholfaden 12 gemäß Fig. 10 vorzugsweise asymmetrisch aufgenäht. Hierdurch werden die Seiten zusammengehalten. Gleichzeitig wird erreicht, daß bei partiellem Einschlag 9 im Innern des Tampons ein Minimum an Hüllmaterial 2 liegt. Das Verpressen zum Tampon erfolgt anschließend wieder in üblicher axialer Richtung, so daß auch hierbei eine Vollumhüllung des entstehenden Tampons gewährleistet wird.

Während bei den bisher beschriebenen Verfahrensschritten Tampons mit im wesentlichen gleichmäßig verteiltem Saugmaterial entstehen, läßt sich durch Veränderung, z. B. durch 90°-Umlenken, des umhüllten Wattebandabschnitts 4, auch ein Tampon mit am Kopf und/oder am Ende relativ mehr Watte-Inhalt als im übrigen Tampon — selbstverständlich bei Beibehaltung der Vollumhüllung — herstellen. Die Abmessungen des einzusetzenden Wattebandabschnitts 4 müssen dabei natürlich den Erfordernissen angepaßt werden. Entsprechende Verfahrensabschnitte werden in den Fig. 11 bis 19 schematisch dargestellt. Im wesentlichen geht es darum, den Rückholfaden 18 nicht parallel sondern im wesentlichen senkrecht zu den eingeschlagenen freien Schnittkanten 5 und 6 auf den umhüllten Wattebandabschnitt 4 aufzubringen. Dazu ist vor dem Befestigen des Rückholfadens 18 ein weiteres Umlenken des Wattebandabschnitts 4 vor dem Einlauf in die zum Aufbringen des Rückholfadens benutzte Maschine, insbesondere Nähmaschine, erforderlich.

Wenn von einem an den freien Schnittkanten 5 und 6 umgeklappten, umhüllten Wattebandabschnitt 4 gemäß Fig. 11 ausgegangen wird, kann dieser zunächst (ähnlich wie in Fig. 9) zusammengefaltet werden, so daß das Zwischenprodukt nach Fig. 12 entsteht. Auf dieses wird nun der Rückholfaden 18 entweder so aufgenäht, daß er gemäß Fig. 13 über die Faltungslinie 17 übersteht oder gemäß Fig. 15 über die Faltungslinie 7 bzw. 8 übersteht. Nach dem Verpressen entsteht dann entweder ein Tampon mit Sicherheitszone 20 nach Fig. 14 am Tamponkopf oder ein Tampon mit Sicherheitszone 21 am unteren Ende gemäß Fig. 16.

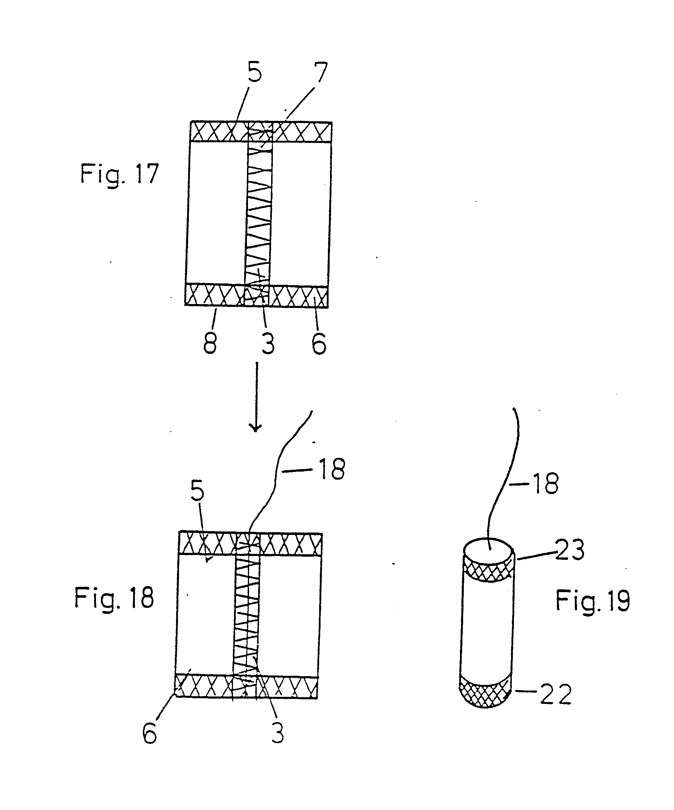

Wird dagegen das Falten des Wattebandabschnitts 4 am Übergang zwischen Fig. 11 und 12 weggelassen und gemäß Fig. 17 auf den ungefalteten aber an den freien Schnittkanten 5 und 6 umgeklappten, umhüllten Wattebandabschnitt 4 der Rückholfaden 18 — etwa parallel zur Überlapplinie 3 — gemäß Fig. 18 aufgebracht, so entsteht nach Fig. 19 ein Tampon mit Watteverstärkung sowohl am Tamponkopf 22 als auch am Tamponende 23.

Bezugszeichenliste

1 = Watteband
2 = Hüllmaterial
3 = Überlapplinie
4 = Wattebandabschnitt
5, 6 = freie Schnittlinie
7, 8 = Faltlinien
9 = Einschlag
10 = Abstand 7/8
11 = Länge
12 = Rückholfaden
13 = Pfeilrichtung
14 = Rundbacken
15 = W-Faltung
16 = Tampon
17 = Faltlinie
18 = Rückholfaden
20, 21 = Sicherheitszone
22 = Tamponkopf
23 = Tamponende

**Patentanspruch**

Verfahren zum Herstellen von Tampons (16) für die Frauenhygiene, bei dem Watteband (1) mit einem flusenfreien Hüllmaterial (2) überlappend umhüllt wird, das Hüllmaterial (2) und das Watteband (1), zur Bildung von umhüllten Wattebandabschnitten (4) mit freien Schnittkanten (5, 6), senkrecht zur Überlappungslinie (3) des Hüllmaterials (2) durchtrennt werden und jeder umhüllte Wattebandabschnitt (4) mit einem parallel oder senkrecht zur Überlappungslinie (3) aufgebrachten Rückholfaden (12, 18) ausgestattet und mittels Rundbacken (14) mit Bezug auf die Überlappungslinie (3) radial oder axial in W-förmiger Faltung (15) verpreßt wird, dadurch gekennzeichnet, daß der umhüllte Wattebandabschnitt (4) nach dem Abtrennen von dem Watteband (1) und vor dem Verpressen und Aufbringen des Rückholfadens (12, 18) in einer Richtung senkrecht zur Längsrichtung des Wattebands (1) zu einer Einrichtung mit Faltungsblechen weitertransportiert wird, daß die nach dem Abtrennen des umhüllten Wattebandabschnitts (4) verbleibenden, senkrecht zu der Überlappungslinie (3) verlaufenden, freien Schnittkanten (5, 6) mit Hilfe der Faltungsbleche auf die Fläche des umhüllten Wattebandabschnitts (4) gefaltet sowie durch Kalanderwalzen angepreßt werden und daß das abschließende Falten und Verpressen so erfolgt, daß die eingefalteten Schnittkanten (5, 6) im Innern des Tampons (16) liegen.

**Claim**

Method of manufacturing tampons (16) for the feminine hygiene, in which cotton wool tape (1) is overlappingly wrapped around by a fluff-free wrapping material (2), the wrapping material (2) and the cotton wool tape (1) are severed through perpendicularly to the overlapping line (3) of the wrapping material (2) for the formation of wrapped cotton wool tape portions (4) with free cutting edges (5, 6) and each wrapped cotton wool tape portion (4) is equipped with a retraction thread (12, 18) mounted parallelly or perpendicularly to the overlapping line (3) and compressed radially or axially in W-shaped folding (15) with respect to the overlapping line (3) by means of round cheeks (14), characterised thereby, that the wrapped cotton wool tape portion (4), after the severing from the cotton wool tape (1) and before the pressing and mounting of the retraction thread (12, 18), is transported further perpendicularly to the longitudinal direction of the cotton wool tape (1) towards an equipment with metal folding plates, that the free cutting edges (5, 6), which remain after the severing of the wrapped cotton wool tape portion (4) and extend perpendicularly to the overlapping line (3), are folded with the aid of the metal folding plates onto the surface of the wrapped cotton wool tape portion (4) as well as pressed on by calender rollers and that the concluding folding and pressing so takes place that the folded-in cutting edges (5, 6) lie in the interior of the tampon (16).

**Revendication**

Procédé pour la fabrication de tampons (16) pour l'hygiène féminine, dans lequel on enveloppe avec chevauchement de la bande d'ouate (1) avec une matière d'enveloppe (2) exempte de peluches, puis on coupe cette bande d'ouate (1) et la matière d'enveloppe (2) pour constituer des tronçons de bande d'ouate enveloppés (4) à bords de coupe libres (5, 6), perpendiculairement à la ligne de chevauchement (3) de la matière d'enveloppe (2), ensuite on équipe chaque tronçon de bande d'ouate enveloppé (4) d'un fil de rappel (12, 18) appliqué parallèlement ou perpendiculairement à la ligne de chevauchement (3), et enfin on comprime chaque tronçon au moyen de mâchoires rondes (14), radialement ou axialement par rapport à la ligne de chevauchement (3), en un pliage en W (15), ce procédé étant caractérisé par le fait qu'après avoir séparé de la bande d'ouate (1) chaque tronçon de bande enveloppe (4) et avant sa compression et l'application du fil de rappel (12, 18), on achemine ce tronçon dans une direction perpendiculaire à la direction longitudinale de la bande d'ouate (1), à un dispositif pourvu de plaques de pliage, ou à l'aide de bandes de pliage on replie sur la surface du tronçon (4) les bords de coupe libres (5, 6), dirigés perpendiculairement à la ligne de chevauchement (3), subsistant après la séparation du tronçon (4) et on presse sur ces bords repliés au moyen de cylindres de calandrage, ce repliage et la compression finale étant effectués de façon que les bords de coupe repliés (5, 6) soient situés à l'intérieur du tampon.

Fig. 4

Fig. 3

Fig. 2

Fig. 1

**0 105 428**

Fig. 5

Fig. 6

Fig. 7

Fig. 8

Fig. 9

Fig. 10

Druck

Fig.14

Fig.13

Fig.11

Fig.12

Fig.16

Fig.15

3

Fig. 17

5   7

8   3   6

—18

Fig. 18

5

6   3

Fig. 19

—18

23

22

4